# FASCICULE DE BREVET EUROPEEN

(11) **EP 1 401 397 B1**
(45) Date de publication et mention de la délivrance du brevet: **09.03.2005**
(21) Numéro de dépôt: 02764922.7
(22) Date de dépôt: 21.06.2002
(51) Int. Cl.: A61K 9/00, A61K 9/16

(54) **FORMULATION PHARMACEUTIQUE AU GOUT MASQUE ET SON PROCEDE DE PREPARATION**
PHARMAZEUTISCHE FORMULIERUNG MIT ÜBERDECKTEM GESCHMACK UND VERFAHREN ZU IHRER HERSTELLUNG
PHARMACEUTICAL FORMULATION HAVING A MASKED TASTE AND METHOD FOR THE PRODUCTION THEREOF

(30) Priorité: 21.06.2001 FR 0108157
(43) Date de publication de la demande: 31.03.2004
(73) Titulaire: Aventis Pharma S.A., 92165 Antony Cédex (FR)
(72) Inventeur: BECOURT, Philippe, F-91300 Massy (FR); CHAUVIN, Josiane, F-77230 Montge en Goele (FR); SCHWABE, Detlev, F-92380 Garches (FR)
(86) Numéro de dépôt international: PCT/FR2002/002158
(87) Numéro de publication internationale: WO 2003/000225

(56) Documents cités:
- EP-A- 0 293 885
- WO-A-99/17742
- US-A- 5 084 278
- DATABASE WPI Section Ch, Week 198831 Derwent Publications Ltd., London, GB; Class A96, AN 1988-215492 XP002191777 & JP 63 150220 A (DAINIPPON PHARM CO LTD), 22 juin 1988 (1988-06-22)

## Description

La présente invention a pour objet une formulation pharmaceutique se présentant sous la forme d'une poudre destinée à l'administration en suspension aqueuse par la voie orale, ayant le goût masqué, son procédé de préparation ainsi qu'une méthode de masquage du goût de produits pharmaceutiques.

Il est bien connu que de nombreux produits pharmaceutiques destinés à être administrés par la voie orale ont un goût très désagréable et parfois une rémanence très grande. C'est notamment le cas des produits antibiotiques désignés communément sous le nom de macrolides ou macrolides-like, comme les kétolides, de céphalosporines ou de quinolones. Ceci revêt bien entendu une importance particulière dans le cas où lesdits produits pharmaceutiques sont destinés à être administrés aux enfants.

De multiples procédés de masquage de goût des produits pharmaceutiques ont été décrits dans la littérature. Le plus souvent, ces procédés consistent à recouvrir les microparticules des produits d'un film qui disparaît après le passage dans la bouche. On peut citer par exemple l'article de Roy "Taste masking in oral pharmaceuticals", Pharmaceutical Technology - April 1994 - p. 84 - 99 ou la demande de brevet WO 98/14179. Il existe également des procédés dans lesquels les matières actives sont incluses dans des matières permettant leur libération dans le corps dans des conditions déterminées. De telles matrices sont décrites par exemple par C. Brossard dans Actualités Pharmaceutiques - N° 388 - Juillet-Août 2000, ou dans les demandes de brevets WO 99/08660, WO 99/17742 ou EP 1027887.

Un problème à résoudre dans le cas d'une matrice est de faire en sorte que la biodisponibilité de la matière active incluse dans une matrice demeure acceptable, compte tenu que les composants (hors principe actif) de la matrice que l'on peut être amené à utiliser ne sont pas nécessairement favorables, pour diverses raisons qui leur sont propres.

L'invention a pour objet une formulation pharmaceutique au goût masqué et dont le masquage persiste lors de l'administration de la formulation, notamment sous forme de suspension dans un véhicule aqueux, caractérisée en ce qu'elle renferme au moins les éléments suivants :
a) - un polymère cellulosique soluble dans des solvants organiques mais pratiquement insoluble dans l'eau quelque soit le pH ;
   - un polymère méthacrylique soluble en milieu acide et pratiquement insoluble à pH neutre ou alcalin et
   - un principe actif réparti de manière homogène et à l'état moléculaire dans le mélange, lequel se présente sous la forme d'une matrice atomisée ;
b) un agent alcalin de nature organique, ou un sel alcalin, pharmaceutiquement acceptable ;
c) un agent adsorbant.

Comme indiqué plus haut, la formulation selon l'invention est destinée à être mise en suspension dans l'eau puis, le cas échéant, stockée en l'état pendant quelques jours, en pratique de 5 à 10 jours, pendant sa période d'absorption.

Il est important de noter que chacun des trois éléments de la formulation coopère avec les deux autres, mais que la totalité des trois éléments est toutefois indispensable pour obtenir l'effet de masquage de goût recherché, lors de l'absorption de la formulation selon l'invention.

De manière non limitative, les différents constituants de la formulation selon l'invention, lesquels doivent être, bien évidemment, pharmaceutiquement acceptables, peuvent être choisis comme suit :
Le polymère cellulosique est notamment l'éthylcellulose. Il doit être soluble dans les solvants organiques mis en oeuvre dans le procédé selon l'invention et aussi insoluble dans l'eau que possible, quel que soit le pH.

Le polymère méthacrylique doit être soluble dans l'eau à pH acide (5 ou inférieur à 5) et aussi insoluble que possible à pH neutre et alcalin, et sa fonction est, dans un premier temps, de faire en sorte que seule la proportion relativement faible de principe actif qui se trouve à la surface des microparticules constituant la formulation, soit accessible à la dissolution après mise en suspension dans l'eau en vue de l'absorption et, dans un second temps, d'assurer une bonne biodisponibilité du principe actif après son ingestion. Il est notamment choisi parmi les polymères connus sous l'appellation Eudragit E et disponibles auprès de la firme Rohm Pharma GmbH, lesquels sont des polymères cationiques formés à partir de 2-diméthylaminoéthyl methacrylate et de méthacrylates neutres.

L'agent alcalin assure un pH alcalin à la formulation après la mise en suspension de celle-ci dans l'eau en vue de son absorption et fait en sorte que la majeure partie du principe actif dispersé dans la matrice, demeure inaccesible à la dissolution après mise en suspension dans l'eau. Ce faisant il contribue à limiter le goût désagréable. Il est notamment choisi dans le groupe constitué par la méglumine, la lysine, le citrate et le carbonate de sodium et de potassium.

L'agent adsorbant fait en sorte d'adsorber à sa surface les quantités du principe actif libérées après la mise en suspension de la formulation dans l'eau tout en permettant sa libération dans l'estomac et donc assurant sa bonne biodisponibilité. Ce faisant, il contribue également à limiter le goût désagréable. Il est notamment choisi parmi le talc et le silicate de magnesium et d'aluminium, ce dernier étant plus particulièrement préféré, notamment dans une granulométrie fine.

Le principe actif peut notamment être un antibiotique de type macrolide ou macrolide-like, c'est-à-dire l'érythromycine et ses dérivés tels que par exemple la roxithromycine, la télithromycine, l'azithromycine, la clarithromycine, ou de type céphalosporine, pénicilline, tétracycline, ou quinolone, mais aussi tout autre type de substance administrée par voie orale et possédant un goût désagréable que l'on veuille masquer, pour autant que sa solubilité dans le solvant organique utilisé dans la préparation de la formulation soit suffisante.

L'invention a particulièrement pour objet une formulation telle que définie précédemment, caractérisée en ce que le principe actif est un antibiotique de type macrolide ou macrolide-like tel que ceux mentionnés plus haut, et, plus particulièrement, un kétolide tel que la télithromycine. Ce composé est décrit par exemple dans le brevet européen EP 0 680 967.

Le polymère cellulosique est présent dans la matrice atomisée dans une proportion qui, de préférence, va de 30 % à 50 % en poids et le polymère méthacrylique dans une proportion de 10 % à 25 % en poids.Le principe actif est présent dans la matrice atomisée à hauteur de 50 % en poids, au maximum.

L'invention a plus particulièrement pour objet une formulation telle que définie précédemment, caractérisée en ce que les proportions de polymères cellulosique et méthacrylique dans la matrice vont respectivement de 40 % à 45 % et de 15 % à 20 % en poids, et en ce que celle du principe actif dans la matrice va jusqu'à 30 % en poids.

Il est admis que pour un produit tel que la télithromycine, une concentration de 10 µl/ml est déjà inacceptable en raison de son goût désagréable et de sa rémanence très grande. Ainsi, compte-tenu de la quantité importante de principe actif se trouvant à la surface des microparticules de la matrice, et qui, par conséquent, est accessible à la dissolution, soit une quantité très largement supérieure aux 10 µl/ml ci-dessus, il est surprenant qu'un masquage du goût soit déjà obtenu par simple "emprisonnement" dans la matrice.

La formulation selon l'invention peut en outre renfermer un ou plusieurs autres éléments connus séparément de l'homme du métier, en particulier un agent plastifiant hydrophobe et un agent anti-oxydant, pour ce qui concerne la matrice elle-même, ainsi qu'un ou plusieurs agents conservateurs, un ou plusieurs agents sucrants, un agent épaississant, ainsi qu'un ou plusieurs agents aromatisants.

Les éléments ci-dessus peuvent notamment être choisis comme suit :
L'agent plastifiant hydrophobe est par exemple, le dibutyl sébacate ou le diéthylphtalate.
L'agent anti-oxydant est, par exemple, l'α-tocophérol, le BHT ou 2,6-di-tert-butyl-4-méthylphénol ou le BHA ou 2-tert-butyl-4-méthoxyphénol.
L'agent conservateur est, par exemple, le méthyl ou le propyl parahydroxybenzoate.
Le ou les agents sucrants sont choisis parmi ceux qui sont communément utilisés dans l'industrie pharmaceutique ou alimentaire, par exemple, le maltitol, le saccharinate de sodium ou le saccharose.
L'agent épaississant est, par exemple, une gomme xanthane ou le sel de sodium de carboxy methyl cellulose.
Le ou les agents aromatisants sont choisis parmi ceux qui sont communément utilisés dans l'industrie pharmaceutique ou alimentaire.

Il est connu qu'un composé tel que l'éthylcellulose qui est insoluble dans l'eau, quelque soit le pH, lorsqu'il est présent, à l'intérieur d'une matrice, réduit la biodisponibilité du principe actif en entravant sa diffusion vers les muqueuses où il est absorbé.

Il est également connu qu'un agent adsorbant tel que le silicate de magnesium et d'aluminium réduit également la biodisponibilité d'un principe actif, en raison du fort effet d'adsorption se manifestant à sa surface (voir par exemple Handbook of Pharmaceutical Excipients p. 269-273,7, 11 et 12 (1994)).

L'homme au métier ne devrait donc pas être incité à utiliser de tels composés pour formuler un principe actif dans les conditions de l'invention.

L'effet bénéfique de masquage de goût obtenu au travers de l'utilisation dans des formulations pharmaceutiques de tels composés devrait donc en toute logique être affecté négativement par la biodisponibilité réduite du principe actif et l'on devrait donc s'attendre à ce que la formulation selon l'invention présente un effet retard en ce qui concerne l'efficacité thérapeutique du principe actif. En fait, tel n'est pas le cas et, de manière inattendue, la formulation démontre au contraire une très bonne biodisponibilité in vivo du principe actif tout en présentant un masquage du mauvais goût dans des proportions tout à fait remarquables.

Ainsi, il a été trouvé dans des tests in vivo que la biodisponibilité peut aller de 60 à 100 % de ce qu'elle est dans le cas des comprimés traditionnels, voire être meilleure dans une proportion pouvant atteindre environ 30 %.

L'invention a également pour objet un procédé de préparation d'une formulation pharmaceutique telle que définie précédemment, caractérisé en ce que l'on mélange au sein d'un solvant organique un polymère cellulosique et un polymère méthacrylique tels que définis plus haut, et, le cas échéant, un agent plastifiant et un agent anti-oxydant tels que définis plus haut, ajoute ensuite le principe actif, puis passe la solution obtenue dans un atomiseur pour obtenir une poudre se présentant sous la forme d'une matrice atomisée, mélange ladite poudre avec un agent alcalin et un agent adsorbant tels que définis plus haut, et, le cas échéant, avec un ou plusieurs éléments choisis dans le groupe constitué par les agents conservateurs, les agents sucrants, les agents épaississants et les agents aromatisants, tels que définis plus haut et obtient la formulation attendue.

Le solvant organique est choisi de telle manière qu'il soit un bon solvant à la fois du polymère cellulosique, du polymère méthacrylique, du principe actif et, le cas échéant, des agents plastifiant et anti-oxydant. Ainsi, on peut notamment utiliser des solvants tels que les hydrocarbures halogénés, notamment le chlorure de méthylène, les alcools, notamment l'éthanol et l'isopropanol et les cétones, notamment l'acétone et la méthyl éthyl cétone.

L'invention a encore pour objet un procédé de masquage du goût d'un principe actif pharmaceutique destiné à l'administration en suspension aqueuse par voie orale, caractérisé en ce que l'on emprisonne de manière homogène ledit principe actif à l'intérieur d'une matrice constituée d'au moins un polymère cellulosique et un polymère méthacrylique tels que définis précédemment et y associe au moins un agent alcalin et un agent adsorbant tels que définis précédemment.

L'exemple suivant illustre l'invention sans toutefois la limiter :

### 1/ Préparation d'une solution pour atomisation

On utilise au départ les ingrédients suivants :

| Composition | g | 1 lot (g) |
|---|---|---|
| Télithromycine | 30.0 | 1050.0 |
| Eudragit E 100® | 18.0 | 630.0 |
| Ethylcellulose N10 | 44.0 | 1540 |
| Dibutyl sebacate | 7.50 | 262.5 |
| α-tocophérol | 0.50 | 17.5 |
| Chlorure de méthylène q.s.p | 1000 | 35000 |

On opère comme suit :
Dans 20 litres de chlorure de méthylène sous agitation modérée, on ajoute le dibutyl sébacate, l' α-tocophérol, l'Eudragit E 100® et l'éthylcellulose N10. On maintient l'agitation toute une nuit.
Une heure avant le début de l'atomisation, on solubilise la télithromycine dans la solution de polymères et complète au poids désiré avec le chlorure de méthylène.

### 2/ Atomisation

L'opération est réalisée dans un atomiseur, selon les paramètres suivants :
Température d'entrée : 85°C
Température de sortie : 45-55°C
Buse bifluide ou monofluide

La buse et la chambre de dessiccation sont placées entièrement sous azote.

### 3/ Séchage secondaire

L'atomisat est étalé sur des plateaux et placé dans une étuve sous flux d'azote à température ambiante pendant 24 heures. On obtient en définitive un atomisat à 30 % de principe actif.

### 4/ Préparation du mélange final

| Composition | G/flacon | Soit pour 2000 flacons (g) |
|---|---|---|
| Atomisat à 30% de télithromycine | 8,334 | 16668 |
| Méglumine | 0,400 | 800 |
| Veegum (F) ® (1) | 2,000 | 4000 |
| Rhodigel ultra ® (2) | 0,025 | 50 |
| Maltitol P 90 (3) | 13,526 | 27052 |
| p.hydroxybenzoate de méthyle | 0,100 | 200 |
| p.hydroxybenzoate de propyle | 0,015 | 30 |
| Saccharinate de sodium | 0,300 | 600 |
| Arôme fraise (05) | 0,300 | 600 |

| | | |
|---|---|---|
| (1) Silicate de magnésium et d'aluminium de granulométrie fine. | | |
| (2) Gomme xanthane. | | |
| (3) P 90 désigne une granulométrie particulière du maltitol. | | |

On utilise un mélangeur dit "par retournement".
Avant d'introduire les produits dans le mélangeur, on les passe dans un tamis ayant une ouverture de maille de 850 µm. On réalise ensuite le mélange comme suit :
- prémélange : tous les excipients et seulement 1/3 du maltitol sont mélangés pendant 10 minutes à 20t/mn ;
- mélange : on ajoute au prémélange le reste de maltitol et l'atomisat, puis mélange pendant 30 minutes à 20 t/mn.
   Le mélange final est ensuite réparti dans les flacons. Il est à noter que les appellations Eudragit E, Veegum F et Rhodigel ultra sont des marques déposées.

## Revendications

1. Une formulation pharmaceutique au goût masqué et dont le masquage persiste lors de l'administration de la formulation, notamment sous forme de suspension dans un véhicule aqueux, **caractérisée en ce qu'**elle renferme au moins les éléments suivants:
a) - un polymère cellulosique soluble dans des solvants organiques mais pratiquement insoluble dans l'eau quelque soit le pH ;
- un polymère méthacrylique soluble en milieu acide et pratiquement insoluble à pH neutre ou alcalin et
- un principe actif réparti de manière homogène et à l'état moléculaire dans le mélange, lequel se présente sous la forme d'une matrice atomisée ;
b) un agent alcalin de nature organique ou un sel alcalin, pharmaceutiquement acceptable ;
c) un agent adsorbant.

2. Une formulation selon la revendication 1, **caractérisée en ce que** le polymère cellulosique et le polymère méthacrylique sont respectivement l'éthylcellulose et un polymère cationique formé à partir de 2-diméthylaminoéthyl méthacrylate et de méthacrylates neutres.

3. Une formulation selon la revendication 1 ou 2, **caractérisée en ce que** l'agent alcalin et l'agent adsorbant sont choisis respectivement dans le groupe constitué par la méglumine, la lysine, les citrates de sodium et de potassium, et les carbonates de sodium et de potassium, et dans le groupe constitué par le silicate de magnésium et d'aluminium et le talc.

4. Une formulation selon la revendication 3, **caractérisée en ce que** l'agent alcalin et l'agent adsorbant sont respectivement la méglumine et le silicate de magnésium et d'aluminium.

5. Une formulation selon l'une quelconque des revendications 1 à 4, **caractérisée en ce que** le principe actif est un antibiotique de type macrolide ou macrolide-like, céphalosporine, pénicilline, tétracycline ou quinolone.

6. Une formulation selon la revendication 5, **caractérisée en ce que** le principe actif est un kétolide.

7. Une formulation selon la revendication 5, **caractérisée en ce que** le principe actif est la télithromycine.

8. Une formulation selon l'une quelconque des revendications 1 à 7, **caractérisée en ce que** :
- le polymère cellulosique est présent dans la matrice atomisée dans une proportion allant de 30 % à 50 % en poids et le polymère méthacrylique, dans une proportion allant de 10 % à 25 % en poids, et
- le principe actif est présent dans la matrice atomisée à hauteur de 50 % en poids, au maximum.

9. Une formulation selon la revendication 8, **caractérisée en ce que** les proportions de polymères cellulosique et méthacrylique dans la matrice vont respectivement de 40 % à 45 % et de 15 % à 20 % en poids, et **en ce que** celle du principe actif est de 30 % en poids, au maximum.

10. Une formulation selon l'une quelconque des revendications 1 à 9, **caractérisée en ce qu'**elle renferme en outre au sein de la matrice, un agent plastifiant hydrophobe et / ou un agent anti-oxydant.

11. Une formulation selon l'une quelconque des revendications 1 à 10, **caractérisée en ce qu'**elle renferme en outre un ou plusieurs éléments choisis parmi les agents conservateurs, les agents sucrants, les agents épaississants et les agents aromatisants.

12. Procédé de préparation d'une formulation pharmaceutique telle que définie à l'une quelconque des revendications 1 à 11, **caractérisé en ce que** l'on mélange au sein d'un solvant organique un polymère cellulosique et un polymère méthacrylique, tels que définis à la revendication 1, et, le cas échéant, un agent plastifiant hydrophobe et un agent anti-oxydant, ajoute ensuite le principe actif, puis passe la solution obtenue dans un atomiseur pour obtenir un produit se présentant sous la forme d'une matrice atomisée, mélange ladite poudre avec un agent alcalin et un agent adsorbant, et, le cas échéant, avec un ou plusieurs éléments choisis dans le groupe constitué par les agents conservateurs, les agents sucrants, les agents épaississants et les agents aromatisants et obtient la formulation attendue.

13. Procédé selon la revendication 12, **caractérisé en ce que** le solvant organique utilisé est choisi dans le groupe constitué par les hydrocarbures halogénés, les alcools et les cétones.

14. Procédé selon la revendication 13, **caractérisé en ce que** le solvant est le chlorure de méthylène.

15. Procédé selon la revendication 12, **caractérisé en ce que** le principe actif est un antibiotique de type macrolide ou macrolide-like, ou de type céphalosporine, pénicilline, tétracycline ou quinolone.

16. Procédé selon la revendication 15, **caractérisé en ce que** le principe actif est un kétolide.

17. Procédé selon la revendication 15 ou 16 **caractérisé en ce que** le principe actif est la télithromycine.

18. Procédé selon la revendication 12, **caractérisé en ce que** l'agent alcalin et l'agent adsorbant sont choisis respectivement dans le groupe constitué par la méglumine, la lysine, le citrate et le carbonate de sodium et de potassium, et dans celui constitué par le talc et le silicate de magnésium et d'aluminium.

19. Procédé de masquage du goût d'un produit pharmaceutique destiné à l'administration en suspension aqueuse par voie orale, **caractérisé en ce que** l'on emprisonne de manière homogène ledit principe actif à l'intérieur d'une matrice constituée d'au moins un polymère cellulosique et un polymère méthacrylique tels que définis à la revendication 1 et y associe au moins un agent alcalin tel que défini à la revendication 1 et un agent adsorbant.

## Patentansprüche

1. Eine pharmazeutische Formulierung mit maskiertem Geschmack, dessen Maskierung bei der Verabreichung der Formulierung, insbesondere in Form einer Suspension in einem wässrigen Träger, anhält, **dadurch gekennzeichnet, dass** sie wenigstens die folgenden Bestandteile umfasst:
a) - ein Cellulosepolymer, das in organischen Lösungsmitteln löslich ist, aber in Wasser praktisch unlöslich ist, wie auch immer der pH sein mag;
- ein Methacrylpolymer, das in saurem Medium löslich ist und bei neutralem oder alkalischem pH praktisch unlöslich ist und
- einen Wirkstoff, der homogen und in molekularem Zustand in dem Gemisch, das in Form einer zerstäubten Matrix vorliegt, verteilt ist;
b) ein pharmazeutisch unbedenkliches alkalisches Mittel organischer Art oder ein Alkalisalz;
c) ein Adsorptionsmittel.

2. Eine Formulierung gemäß Anspruch 1, **dadurch gekennzeichnet, dass** das Cellulosepolymer und das Methacrylpolymer jeweils Ethylcellulose und ein aus 2-Dimethylaminoethylmethacrylat und neutralen Methacrylaten gebildetes kationisches Polymer sind.

3. Eine Formulierung gemäß Anspruch 1 oder 2, **dadurch gekennzeichnet, dass** das alkalische Mittel und das Adsorptionsmittel jeweils ausgewählt sind aus der Gruppe, die aus Meglumin, Lysin, Natrium- und Kaliumcitrat und Natrium- und Kaliumcarbonat besteht, und aus der Gruppe, die aus Magnesium- und Aluminiumsilikat und Talk besteht.

4. Eine Formulierung gemäß Anspruch 3, **dadurch gekennzeichnet, dass** das alkalische Mittel und das Adsorptionsmittel Meglumin beziehungsweise Magnesiumund Aluminiumsilikat sind.

5. Eine Formulierung gemäß einem der Ansprüche 1 bis 4, **dadurch gekennzeichnet, dass** der Wirkstoff ein Antibiotikum vom Typ Makrolid oder Makrolid analog, Cephalosporin, Penicillin, Tetracyclin oder Chinolon ist.

6. Eine Formulierung gemäß Anspruch 5, **dadurch gekennzeichnet, dass** der Wirkstoff ein Ketolid ist.

7. Eine Formulierung gemäß Anspruch 5, **dadurch gekennzeichnet, dass** der Wirkstoff Telithromycin ist.

8. Eine Formulierung gemäß einem der Ansprüche 1 bis 7, **dadurch gekennzeichnet, dass**:
- das Cellulosepolymer in der zerstäubten Matrix in einem Anteil von 30 bis 50 Gew.-% vorhanden ist und das Methacrylpolymer in einem Anteil von 10 bis 25 Gew.-% und
- der Wirkstoff in der zerstäubten Matrix in einer Höhe von maximal 50 Gew.-% vorhanden ist.

9. Eine Formulierung gemäß Anspruch 8, **dadurch gekennzeichnet, dass** die Anteile an Cellulose- und Methacrylpolymer in der Matrix von 40 bis 45 Gew.-% beziehungsweise von 15 bis 20 Gew.-% gehen, und dadurch, dass derjenige des Wirkstoffs maximal 30 Gew.-% beträgt.

10. Eine Formulierung gemäß einem der Ansprüche 1 bis 9, **dadurch gekennzeichnet, dass** sie außerdem im Innern der Matrix einen hydrophoben Weichmacher und/oder ein Antioxidationsmittel enthält.

11. Eine Formulierung gemäß einem der Ansprüche 1 bis 10, **dadurch gekennzeichnet, dass** sie außerdem ein oder mehrere Bestandteile enthält, die ausgewählt sind unter den Konservierungsstoffen, den Süßstoffen, den Verdickungsmitteln und den Aromatisierungsmitteln.

12. Verfahren zur Herstellung einer pharmazeutischen Zusammensetzung, wie in einem der Ansprüche 1 bis 11 definiert, **dadurch gekennzeichnet, dass** man in einem organischen Lösungsmittel ein Cellulosepolymer und ein Methacrylpolymer, wie in Anspruch 1 definiert, und gegebenenfalls einen hydrophoben Weichmacher und ein Antioxidationsmittel mischt, dann den Wirkstoff hinzufügt, dann die erhaltene Lösung in einen Zerstäuber gibt, um ein Produkt zu erhalten, das in Form einer zerstäubten Matrix vorliegt,. besagtes Pulver mit einem alkalischen Mittel und einem Adsorptionsmittel und gegebenenfalls mit einem oder mehreren Bestandteilen, die ausgewählt sind aus der Gruppe, die aus den Konservierungsmitteln, den Süßstoffen, den Verdickungsmitteln und den Aromatisierungsmitteln besteht, mischt und die erwartete Formulierung erhält.

13. Verfahren gemäß Anspruch 12, **dadurch gekennzeichnet, dass** das verwendete organische Lösungsmittel ausgewählt ist aus der Gruppe, die aus den halogeniertem Kohlenwasserstoffen, den Alkoholen und den Ketonen besteht.

14. Verfahren gemäß Anspruch 13, **dadurch gekennzeichnet, dass** das Lösungsmittel Methylenchlorid ist.

15. Verfahren gemäß Anspruch 12, **dadurch gekennzeichnet, dass** der Wirkstoff ein Antibiotikum vom Typ Makrolid oder Makrolid analog oder vom Typ Cephalosporin, Penicillin, Tetracyclin oder Chinolon ist.

16. Verfahren gemäß Anspruch 15, **dadurch gekennzeichnet, dass** der Wirkstoff ein Ketolid ist.

17. Verfahren gemäß Anspruch 15 oder 16, **dadurch gekennzeichnet, dass** der Wirkstoff Telithromycin ist.

18. Verfahren gemäß Anspruch 12, **dadurch gekennzeichnet, dass** das alkalische Mittel und das Adsorptionsmittel jeweils ausgewählt sind aus der Gruppe, die aus Meglumin, Lysin, Natrium- und Kaliumcitrat und -carbonat besteht, und aus derjenigen, die aus Talk und Magnesium- und Aluminiumsilikat besteht.

19. Verfahren zur Maskierung des Geschmacks eines pharmazeutischen Produkts, das für die orale Verabreichung in wässriger Suspension bestimmt ist, **dadurch gekennzeichnet, dass** man besagten Wirkstoff im Innern einer Matrix, die aus wenigstens einem Cellulosepolymer und einem Methacrylpolymer, wie in Anspruch 1 definiert, homogen einschließt und wenigstens ein alkalisches Mittel, wie in Anspruch 1 definiert, und ein Adsorptionsmittel damit kombiniert.

## Claims

1. Pharmaceutical formulation with a masked taste, the masking of which persists during the administration of the formulation, especially in the form of a suspension in an aqueous vehicle, **characterized in that** it contains at least the following components:
a) - a cellulose-based polymer that is soluble in organic solvents but virtually insoluble in water irrespective of the pH;
- a methacrylic polymer that is soluble in acidic medium and virtually insoluble at neutral or alkaline pH, and
- an active principle uniformly distributed and in molecular form in the mixture, which is in the form of an atomized matrix;
b) a basic agent of organic nature or a pharmaceutically acceptable basic salt;
c) an adsorbent.

2. Formulation according to Claim 1, **characterized in that** the cellulose-based polymer and the methacrylic polymer are, respectively, ethylcellulose and a cationic polymer formed from 2-dimethylaminoethyl methacrylate and neutral methacrylates.

3. Formulation according to Claim 1 or 2, **characterized in that** the basic agent and the adsorbent are chosen, respectively, from the group consisting of meglumine, lysine, sodium citrate, potassium citrate, sodium carbonate and potassium carbonate, and from the group consisting of magnesium aluminium silicate and talc.

4. Formulation according to Claim 3, **characterized in that** the basic agent and the adsorbent are, respectively, meglumine and magnesium aluminium silicate.

5. Formulation according to any one of Claims 1 to 4, **characterized in that** the active principle is a macrolide or macrolide-like, cephalosporin, penicillin, tetracycline or quinolone antibiotic.

6. Formulation according to Claim 5, **characterized in that** the active principle is a ketolide.

7. Formulation according to Claim 5, **characterized in that** the active principle is telithromycin.

8. Formulation according to any one of Claims 1 to 7, **characterized in that**:
- the cellulose-based polymer is present in the atomized matrix in a proportion ranging from 30% to 50% by weight, and the methacrylic polymer in a proportion ranging from 10% to 25% by weight, and
- the active principle is present in the atomized matrix upto a proportion of 50% by weight.

9. Formulation according to Claim 8, **characterized in that** the proportions of cellulose-based and methacrylic polymers in the matrix range from 40% to 45% and from 15% to 20% by weight, respectively, and **in that** the proportion of the active principle is upto 30% by weight.

10. Formulation according to any one of Claims 1 to 9, **characterized in that** it also contains in the matrix a hydrophobic plasticizer and/or an antioxidant.

11. Formulation according to any one of Claims 1 to 10, **characterized in that** it also contains one or more components chosen from preserving agents, sweeteners, thickeners and flavourings.

12. Process for preparing a pharmaceutical formulation as defined in any one of Claims 1 to 11, **characterized in that** a cellulose-based polymer and a methacrylic polymer as defined in Claim 1 and, where appropriate, a hydrophobic plasticizer and an antioxidant are mixed together in an organic solvent, the active principle is then added, the solution obtained is then placed in an atomizer to obtain a product in the form of an atomized matrix, the said powder is mixed with a basic agent and an adsorbent and, where appropriate, with one or more components chosen from the group consisting of preserving agents, sweeteners, thickeners and flavourings, and the expected formulation is obtained.

13. Process according to Claim 12, **characterized in that** the organic solvent used is chosen from the group consisting of halogenated hydrocarbons, alcohols and ketones.

14. Process according to Claim 13, **characterized in that** the solvent is methylene chloride.

15. Process according to Claim 12, **characterized in that** the active principle is a macrolide or macrolide-like antibiotic or a cephalosporin, penicillin, tetracycline or quinolone antibiotic.

16. Process according to Claim 15, **characterized in that** the active principle is a ketolide.

17. Process according to Claim 15 or 16, **characterized in that** the active principle is telithromycin.

18. Process according to Claim 12, **characterized in that** the basic agent and the adsorbent are chosen, respectively, from the group consisting of meglumine, lysine, sodium and potassium citrate and carbonate, and from the group consisting of talc and magnesium aluminium silicate.

19. Process for masking the taste of a pharmaceutical product for oral administration as an aqueous suspension, **characterized in that** the said active principle is uniformly enclosed within a matrix consisting of at least one cellulose-based polymer and one methacrylic polymer as defined in Claim 1, and at least one basic agent as defined in Claim 1 and an adsorbent are combined therewith.
